# EUROPEAN PATENT SPECIFICATION

(11) **EP 0 328 390 B1**
(45) Date of publication and mention of the grant of the patent: **14.09.1994**
(21) Application number: 89301259.1
(22) Date of filing: 09.02.1989
(51) Int. Cl.: C07K 7/10, C07K 13/00, C12P 21/02, A61K 39/21, C12N 15/00

(54) **Peptide treatment of refractory infectious diseases**
Peptidbehandlung von hartnäckigen Infektionskrankheiten
Traitement aux peptides de maladies infectieuses rebelles

(30) Priority: 10.02.1988 US 154862
(43) Date of publication of application: 16.08.1989
(73) Proprietor: IMMULOGIC PHARMACEUTICAL CORPORATION, Cambridge Massachusetts 02139 (US)
(72) Inventor: Gefter, Malcolm L., Weston, MA 02193 (US)
(74) Representative: Harrison, David Christopher

(56) References cited:
- EP-A- 0 225 066
- EP-A- 0 247 557
- EP-A- 0 311 219
- WO-A-87/06005
- THE JOURNAL OF IMMUNOLOGY, vol. 139, no. 12, 15 December 1987, The American Association of Immunologists, US; M.-Z. LAI et al., pp. 3973-3980#

## Description

Methods and compositions for treating refractory infectious diseases and, in particular, AIDS.

Developing a vaccine to protect against infection by human immunodeficiency virus (HIV) has proven difficult. In addition, although passively-admininstered antibody, such as following exposure to hepatitis virus, or post-infection vaccination, as with rabies virus, has been useful in curing or controlling viral disease in infected individuals for these viral diseases, the treatment of AIDS patients has essentially been limited to treatment of opportunistic infections or other symptoms of the disease resulting from the host's weakened immune system.

One of the problems with developing a vaccine for HIV is that the viruses have been reported to be highly polymorphic, frequently changing surface antigens in a few cycles of replication.

A serious problem for vaccinating HIV-infected patients is that when the virus infects a population of T-cells, it integrates into the genome of the cells and may remain latent. However it appears that upon stimulation of the T-cell as by an opportunistic infection or a vaccine, the virus begins replicating, resulting in death of the infected T-cells. Therefore, if an attenuated strain of HIV or an envelope protein from HIV is administered to an infected patient to induce antibodies, infected T-cells may be stimulated, resulting in replication of the formerly latent virus and death of the infected T-cells.

Although anti-HIV antibody may be useful to prevent the disease in uninfected individuals and to control the disease in infected individuals, the potential viral replication caused by stimulation of infected helper T-cells accompanying vaccine-induced antibody induction presents an additional impediment to the development of safe and effective vaccines.

### Relevant Literature

The various antigens of the retrovirus are described by Saxinger et al., Science (1985) 227:1036-1038. See also Gallo et al., ibid. (1984) 224:500; Saragadharn et al., ibid. 224:506; Barre-Sinoussi et al., ibid. (1983) 220:868; Montagnier et al., in Human T-Cell Leukemia/Lymphoma Virus, Gallo, Essex, Gross, eds. (Cold Spring Harbor Laboratory, Cold Spring Harbor, New York), 1984, p. 363. These may include, but are not limited to p13, p18, p25, p36, gp43, p55, gp65, gp110, etc., where the numbers may differ depending upon the reporter.

The complete nucleotide sequence of LAV is reported by Wain-Hobson et al., Cell (1985) 40:9. The complete sequence for HTLV-III is reported by Muesing et al., Nature (1985) 313:450, while the complete sequence for ARV is reported by Sanchez-Pescador et al., Science (1985) 227:484. All three viruses exhibit substantial nucleotide homology and are similar with respect to morphology, cytopathology, requirements for optimum reverse transcriptase activity, and at least some antigenic properties (Levy et al., Science (1984) 225:840; Shupbach et al., Science (1984) 224:503), and hence should be considered isolates of the same virus which is now usually referred to as HIV. See also, Chang et al., Science (1985) 228:93.

A number of peptides have been reported to bind HIV antibodies as reported by Chang et al., Nature (1985) 315:151-154; Chang et al., Science (1985) 228:93-96 and Barin et al., Science (1985) 228:1094-1096. Kennedy et al., Science (1986) 231:1556-1559 report that antiserum induced by a synthetic peptide recognizes the virus and that anti-viral antibodies bind the peptide. (See also Chem Eng. News (1987) v. 65 which is devoted to reviewing progress in understanding AIDS.

EP 225066, EP 247557 and WO 87/06005 all describe HIV antigen determinant peptides.

Review articles that demonstrate the extra-ordinary progress and rate at which an understanding of T-cell restriction and the immune system has occurred is shown by Berzofsky, The Year in Immunology (1986) 2:28-38; Schwartz, Ann. Rev. Immunol. (1985) 3:237-261; Shastri et al., J. Exp. Med. (1986) 164:882-896. See also Shastri et al., ibid. (1985) 162:332-345; Unanue and Allen, Science (1987) 236:551-557; Guillet et al., Science (1987) 235:865-870.

### SUMMARY OF THE INVENTION

Peptides and compositions for treating a host susceptible to HIV infection are provided as set out in claims 1, 7 and 11. The compositions comprise at least one peptide compound having a first region of not more than about 50 amino acids cross-reactive with an HIV B-cell epitope and a second region comprising an immunodominant sequence restricted by a human haplotype of interest and providing for T-cell stimulation.

The compounds provided can be administered to the host in an amount sufficient to induce antibodies.

### DESCRIPTION OF THE SPECIFIC EMBODIMENTS

Novel peptide compounds are provided, as well as their use in treating a host susceptible to HIV. The peptide compounds induce the production of antibody to HIV while stimulating only a small population of helper T-cells. The peptide compound will have at least two regions, art HIV B-cell epitope and an immunodominant sequence. The peptide compound may comprise the sequence of two different peptides and be prepared by recombinant techniques or chemical synthesis, where the HIV B-cell epitope is a fragment of a naturally-occurring HIV peptide joined to a second peptide which may be a fragment of a naturally-occurring peptide, a wholly synthetic peptide or a combination thereof.

In referring to an epitope, the epitope will be the basic element or smallest unit of recognition by a receptor, particularly immunoglobulins, histocompatibility antigens and T-cell receptors, where the amino acids essential to the receptor recognition may be contiguous and/or non-contiguous in the sequence.

For the most part, the peptides employed in this invention will have at least about 10 amino acids, usually at least 12 amino acids, more usually at least 15 amino acids and fewer than 5,000 amino acids. Desirably, the peptide compound will have fewer than 1,000 amino acids, usually fewer than 500, more usually fewer than 250 amino acids, preferably fewer than about 100 amino acids, and more preferably fewer than about 75 amino acids, generally ranging from about 12 to 60 amino acids. The peptides may be glycosylated or unglycosylated.

The HIV B-cell epitope sequence will be joined to an immunodominant sequence to form a novel peptide where each region will be less than about 80% of the total molecule, usually the immunodominant sequence being from about 10 to 80% and the HIV epitope sequence being from about 20 to 80%. The peptide compound may be a combination of a plurality of regions. Thus the compound may include a plurality of B-cell epitopes and/or may include a plurality of immunodominant sequences.

Usually, the number of different HIV B-cell epitope sequences will range from 1 to 20, more usually from about 1 to 10, conveniently from about 1 to 6. Depending upon the nature of the individual epitopes, the epitopes may be fused without an intervening sequence head-to-tail or may be separated by bridging groups of from about 1 to 30 amino acids, usually from about 1 to 20 amino acids.

The bridging groups may be any convenient sequence, but will usually be subject to selection so as to avoid interference with the proper binding of the epitopic sequences and to avoid creating undesirable immune responses to epitopes which could be detrimental to the host.

The peptide will also comprise at least one immunodominant sequence, usually having not more than four, more usually not more than three. In counting immunodominant sequences is intended a sequence of contiguous amino acids of not more than about 36 amino acids, usually not more than about 30 amino acids. The immunodominant sequence may be specific for a particular haplotype, may include a particular sequence which binds to a plurality of haplotypes, or may have a multiplicity of sequences which bind to the same or different haplotypes. Since about twelve amino acids will occupy the transplantation antigen cleft, binding sequences may overlap or be in tandem in a relatively short peptide sequence. Thus, by selecting an immunodominant sequence which includes an extended sequence which binds to a plurality of haplotypes, one can provide for activity with a large segment of the population due to the heterozygosity of humans.

Usually, the peptide will have not more than 3, more usually not more than 2, different immunodominant regions, particularly differing in being restricted by different haplotypes or groups of haplotypes.

The HIV B-cell epitope region can be joined to the immunodominant sequence region either directly or through a bridge, usually having fewer than about 50 amino acids, more usually having fewer than about 30 amino acids. In this manner, a single fused protein may be obtained, where the immunodominant region(s) is fused to one or more HIV B-cell epitope sequences.

Rather than having a fused protein, one may join the immunodominant region and HIV B-cell epitope sequence by linking the immunodominant region to a peptide comprising an HIV B-cell epitope. The linkage may take a variety of forms, the particular manner of linkage not being critical. Thus, one can provide for a cysteine to be present at a terminus or other site of the peptide, where the B-cell epitope of interest may be functionalized with a maleimide group. By combining the cysteine modified immunodominant sequence with the HIV epitope, a thioether linkage may be achieved. If appropriate, a carboxyl group present on the immunodominant sequence may be activated using carbodiimide, or forming an active ester, e.g. p-nitrophenyl ester, where any available amino groups present on the peptide are blocked. After reacting the peptide with the HIV B-cell epitope of interest to form a peptide bond, the blocking groups may be removed. Other techniques may also be employed, as described by Fieser and Fieser, Reagents for Organic Synthesis, Vol. 3, Wiley-Interscience, NY, 1972.

To stimulate antibody production, one employs a B-cell epitope joined to an immunodominant region restricted by a human haptotype(s) of interest providing for T-cell stimulation. For the most part, the B-cell epitope sequence region will vary from about 6 amino acids, usually at least 10 amino acids, more usually at least 15 amino acids to about 50 amino acids, more usually about 30 amino acids. The HIV B-cell epitope sequences will, for the most part, be less than about 5 kD, usually less than about 3 kD, so that they would normally be considered haptenic.

The B-cell epitope may be any epitope of HIV, particularly of an envelope or other surface protein, desirably a neutralizing epitope. A neutralizing epitope as used herein means an epitope which when bound by antibody results in virus neutralization, i.e. loss of infectivity of the virus. (An antibody which interferes with viral binding to the CD4 protein will neutralize viral infectivity.) The HIV B-cell epitope sequence will be selected to avoid HIV immunodominant sequences restricted by Class II transplantation antigens. The presence of an immunodominant sequence restricted by a Class II transplantation antigen in the peptide can be determined by exposing a culture of human T-cells to the peptide and assaying for inter-leukin-2 (IL-2) production. If IL-2 is produced, the peptide comprises such an immunodominant sequence and is undesirable for use in the present compounds.

One could also employ a plurality of strain-specific and cross-reactive epitopes of the same or different HIV strains. A plurality of B-cells would be stimulated, where the different subsets of B-cells were specific for different epitopic sites. Use of a plurality of HIV B-cell epitopes would be particularly advantageous since HIV coat antigens undergo rapid mutation. Thus, in preparing a vaccine, one could provide for epitopes of different strains of HIV, so that B-cells recognizing all of the different strains would be stimulated at the same time, by virtue of a single formulation.

In selecting a B-cell epitope sequence, one can use any sequence which induces antibodies that bind to the virus. A number of HIV protein sequences have been described in the literature, where particular amino acid sequences are said to be recognized by anti-HIV antibodies produced in infected hosts and are useful in production of antibodies for protection of hosts against HIV. Of particular interest are protein fragments from gp160 which is cleaved to form gp120, the envelope glycoprotein, and gp41, the transmembrane protein which anchors the gp120 to the HIV particle. Barin et al., Science (1985) 228:1094-1096 describes that gp160 and gp120 were the antigens most consistently recognized by antibodies in AIDS patients. A number of other articles describe particular portions of the envelope proteins, and particularly gp160 and its cleavage products which induce antibodies that recognize the native virus. In particular, Kennedy et al. describe using a synthetic protein corresponding to the amino acid sequence of residues 735-752 of gp160. Lasky et al., Cell (1987) 50:975 describe the use of residues 397-439 of gp120. Additionally, the core protein, p17 appears to lie near the lipid membrane. A polypeptide designated HGP-30 (a 30 amino acid protein containing most of the gp17 sequence) appears to induce neutralizing antibody in vitro. (See Baum, Chem. Eng News (1987) 65:27-34 at page 34.) In a preferred embodiment, the HIV B-cell epitope will contain at least 5 consecutive amino acids within residues 735-752 of gp160 or residues 397-439 of gp120.

The B-cell epitope sequence will usually have the same amino acid sequence as a fragment of an HIV protein. However, sequences having 1 to 3, usually 1 to 2, more usually 1, mutation(s) which sequences are cross-reactive with HIV B-cell epitope sequences may also find use. By cross-reactive is meant that antibodies induced by the peptide sequence recognize and bind to the HIV epitope and vice-versa. The terms "mutation" or "lesion" as used herein mean a deletion, insertion or substitution of one amino acid in a sequence. Cross-reactive, mutated sequences can be produced using conservative substitutions as described later. There will usually be no advantage to using mutated sequences so that for the most part the HIV B-cell epitope sequences will be identical to a fragment of an HIV protein of a particular strain. Desirably, the fragment will be from a conserved region. The HIV fragment sequence may be glycosylated or unglycosylated, to evoke an immune response to the protein. (See Baum, supra at page 33.)

The second region is an immunodominant region, which may include a contiguous sequence from a naturally occurring protein capable of binding to one or a plurality of haplotypes or non-contiguous regions of a naturally occurring protein, where each region may be able to bind to one or more haplotypes. That is, the immunodominant sequence may be comprised of a single agretope or a plurality of agretopes, where different agretopes may or may not employ common amino acids. The agretopes may be overlapping, contiguous or separated by an amino acid sequence. Alternatively, the immunodominant region may be synthesized to provide a sequence which does not have a known analogy among naturally occurring proteins.

The immunodominant sequence will comprise agretope(s) which will be restricted by human Class II transplantation antigens and T-cell receptor epitopes, which are recognized by a subset of helper T-cells. (By "recognized," is intended bound in the environment--including the cleft of the transplantation antigen--of the Class II HLA.) The choice of the immunodominant sequence may be varied widely, being selected in accordance with a number of considerations. Where the protein is to be used for a large population of different haplotypes, it will be desirable to select sequences which are restricted by a large number of haplotypes. Thus, one would select one or more immunodominant sequences which are poly-restricted (bind to a plurality of different haplotypes).

Normally, the immunodominant sequence region will be a heterologous (non-HIV) sequence which is normally different from an immunodominant sequence of an HIV protein. However, the immunodominant sequence region may be an immunodominant sequence of an HIV protein or a sequence cross-reactive therewith joined to the HIV B-cell epitope by other than the natural sequence. Desirably, the subject immunodominant sequence will not usually cause stimulation of the same T-cell subsets stimulated by HIV proteins. When the immunodominant sequence is a naturally-occurring protein, the immunodominant sequence comprises at least 5 amino acids and usually not more than about 75% of the protein. Usually, the immunodominant sequence will comprise not more than 50%, more usually not more than about 25%, preferably not more than about 10% of the heterologous protein sequence.

For the most part, the immunodominant sequence will vary from about 5 amino acids, usually at least 8 amino acids, more usually at least 10 amino acids to not more than about 50 amino acids, usually not more than about 30 amino acids, preferably not more than 18 amino acids. It will be further characterized by being restricted by a human transplantation antigen and capable of stimulating one or more human T-cell subsets.

The amino acid sequence which binds to the transplantation antigen is referred to as the "agretope". The agretope is a single unit of recognition which specifically binds to one or a limited number of transplantation antigens. The agretope is defined by a set of agretopic amino acid residues, with the individual amino acid residues frequently separated. Usually, there will be not more than about 7 amino acids involved with the agretope, more usually not more than about 5 amino acids defining the agretope, normally at least 2 amino acids, more usually at least about 3 amino acids. The amino acids may be in tandem or separated by from 1 to 4 amino acids, usually 2 to 4 amino acids.

At least 3 amino acids interspersed between the agretopic residues may provide the T-cell receptor epitopic recognition sequence, by themselves or in conjunction with one or more of the agretopic residues. The amino acids of the T-cell receptor epitope define which T-cell receptors bind to the complex of the immunodominant peptide and the transplantation antigen.

The human transplantation antigens (HLA) involved in antibody production are Class II: see Figueroa and Klein, Immunology Today (1986) 7:78-81, and references cited therein. The human Class II antigens are divided into DP, DQ and DR. The different transplantation antigens may play different roles in stimulating different classes of T-cells. Hirayama et al., supra, and references cited therein, suggest that helper T-cells (CD4⁺) are restricted by DR. Using that relationship, helper cells may be stimulated by including an agretope which is DR restricted.

The sequence defining the T-cell receptor epitope can be selected to activate a particular subset(s) or population of T-cells. Desirably, the particular T-cell receptor epitope used exhibits substantially no cross-reactivity with immunodominant sequences restricted by Class II transplantation antigens of strains of HIV to which the host may have been exposed, usually not cross-reactive with such immunodominant sequences of any HIV strain. Usually, the immunodominant sequence will share less than about 30% homology with an HIV immunodominant sequence restricted by a Class II haplotype.

Preferably, the T-cell receptor epitope is one which is not likely to have been previously encountered by the host. In this way, T-cells recognizing the epitope will be present as a small percentage of the T-cell population, rather than in expanded numbers due to prior exposure to the receptor. Since the T-cells will be present in small numbers, it is statistically less likely that any of the cells of that small population will be infected, since usually even in AIDS only about 10% of the T-cells are reported to be infected. Further, if the T-cells were not previously stimulated, that population is more likely to be present in an AIDS infected host.

When using vaccines containing a T-cell receptor epitope the host has previously encountered, particularly an HIV T-cell receptor epitope in an infected host, the responsive T-cell population may have been eliminated by stimulation of those T-cells leading to virus replication and cell death. Thus, the T-cell receptor epitope is preferably selected to stimulate a small population of T-cells which are likely to be present. In this way the likelihood of stimulating an antibody response while avoiding inducing viral replication is enhanced.

A plurality of immunodominant sequences restricted by different haplotypes may be present in a peptide compound of this invention. However it is preferred to have the same or similar T-cell receptor epitopes in each immunodominant sequence. Use of a plurality of T-cell receptor epitopes is disadvantageous in that as the number of stimulated T-cells increases, statistically, the likelihood of stimulating infected T-cells increases. For the most part, since most hosts will be heterozygous, if one wishes to involve more than one transplantation antigen allele, it will be desirable to have at least two immunodominant sequences unless the two transplantation antigens have substantially similar consensus sequences.

The immunodominant region may be selected in a variety of ways. One may use a naturally-occurring immunodominant sequence, where the T-cell receptor epitope is selected arbitrarily. Alternatively, the region may be selected so as to have an epitopic site associated with an innocuous T-cell receptor epitope which the host is unlikely to have encountered. For example one may use immunodominant sequences of proteins from phage, aquatic plants, or animals and other sources which the host is unlikely to have encountered. Commonly used foreign proteins for antibody induction include keyhole limpet hemocyanin, bovine gamma-globulin, BCG, ovalbumin, etc.

The immunodominant sequence may also have the agretope of a naturally-occurring immunodominant sequence mutated to enhance the affinity of the immunodominant sequence for one or more transplantation antigens present in the host. Alternatively, the sequence may be a wholly synthetic sequence designed specifically to bind a haplotype of the host. Because of the enhanced binding affinity to host transplantation antigens, an enhanced immune response will be achieved.

The immunodominant sequence of the peptide is defined in relation to the transplantation antigen sequence and the immunogens restricted by the transplantation antigen. The sequence of the transplantation antigen may be determined by comparing homology between the immunodominant sequences of antigens restricted by the transplantation antigen, where included in the comparison may or may not be the polymorphic region of the transplantation antigen. (For a discussion of the immunodominant sequence see, for example, Berzofsky, (1986) supra, and references cited therein.)

Transplantation antigens have polymorphic regions, where the individual alleles are associated with specific hosts. For the most part, the host will be diploid and heterozygous, so that each host will have two haplotypes, meaning that there will be two different copies of a particular transplantation antigen type from the same locus (except where the host is homozygous at that particular locus).

The homology of the immunodominant sequences may be compared using the FASTP algorithim, although any other algorithim which allows for comparison of homology may be employed. For a description of FASTP, see Lipman and Pearson, Science (1985) 227:1435-1441. The immunodominant sequences should have about 30% homology or greater, with up to a total of 20%, usually not more than about 15% based on the number of amino acids in the sequence of deletions or insertions to provide for the desired homology. That is one counts the non-conservative substitutions first, then deletions and insertions to provide the best homology. Desirably, the homology will involve identity, rather than conservative substitutions. Usually, there will be not more than a total of 2 amino acids involved in insertions or deletions, and usually not more than about 1 insertion or deletion, usually deletion.

The following table indicates conservative substitutions, where any amino acid on the same line may be substituted for any other amino acid on the same line.

Amino Acids
Aliphatic
non-polar
G, A, (P)
V, L, I
polar
neutral
C, M, S, T
N, Q
acidic
D, E
basic
K, L, (H)
aromatic
F, W, Y, (H)

Proline (P) may be considered equivalent, but will normally not be substituted for the other amino acids on the same line. Similarly, Histidine (H) may be substituted for the other amino acids on the same line, but will not normally be considered an equivalent. In addition, in some instances, the acidic, basic, and polar amic amino acids (N, Q) may be substituted one for the other in determining homology.

Normally, at least 2 immunodominant sequences will be involved in the determination of a consensus sequence for an agretope, more usually 3 and not more than about 8 should suffice, usually 3 to 6 sequences will suffice. The immunodominant sequences may be identified in a number of ways. Particularly, the protein restricted by the transplantation antigen may be divided into a number of sequences, which may be synthesized and then used in an assay, where cells containing the particular transplantation antigen and T-cells restricted by the transplantation antigen and specific for the specific antigen are combined. By determining the level of secretion of IL-2, one can define the sequence which is immunodominant. Usually there will be a single immunodominant sequence, although in some antigens, there may be more than 1. However, where there is more than 1, both the sequences may be used in defining a consensus sequence for optimizing binding affinity to the particular transplantation antigen.

The compositions of this invention may be a single peptide or a mixture of peptides, usually a mixture of peptides. Generally, the number of peptides will not exceed about 20, more usually not exceed about 12, and preferably not exceed about 8, more preferably not exceed about 6. The mixtures will usually be directed to those transplantation antigens which are most frequently found in the population of interest. That is, particular transplantation antigens may be more frequent in certain population groups of people which can be reflected in the vaccine peptide(s).

The subject peptides may be prepared by any convenient means. Usually, either chemical synthesis will be employed or recombinant techniques. Synthesis methods are well known and include the method of Merrifield which can be performed manually or by commercially available apparatus.

Based on known techniques, one can synthesize genes encoding the subject compositions. Techniques for synthesizing oligodeoxynucleotide single strands are well established and strands may be obtained of 200 bases or more. By appropriately overlapping strands, large synthetic sequences can be prepared. Alternatively, where one has mutated the sequence of an available antigen, various techniques are available for precisely introducing the mutation, such as in vitro mutagenesis, restriction and insertion of a synthetic sequence, or the like. The particular manner in which the mutation is carried out is not critical to this invention.

Once the gene has been obtained it may be used in accordance with conventional techniques for expression. A significant number of expression vectors are available commercially or in the literature, which may be used with advantage in the subject invention. The host transformed with the vectors may provide for stable extrachromosomal maintenance or integration into the host genome. Convenient expression hosts include E. coli, B. subtilis, B. licheniformis, Saccharomyces, e.g. cerevisiae, Kluyveromyces, e.g. lactis. Of primary interest will be mircoorganisms, particularly bacteria and fungi, e.g. yeast.

If desired, the constructs can be prepared in conjunction with known signal leaders for secretion. Signal leaders include the α-factor of yeast, α-amylase, penicillinase, surface membrane proteins, etc. The signal leader with its processing signal may be joined at the 5′ terminus of the gene, so as to provide for a fused precursor, which upon maturation loses the signal leader and processing signal.

The subject compositions may be formulated in a variety of ways for administation to a patient. They may be formulated in any convenient physiologically acceptable medium for administation to a human host. These media include water, saline, phosphate buffered saline, oil emulsions, etc. In some instances it may be desirable to formulate the subject peptides as tablets, microcapsules, e.g. slow release, within or bound to liposomes, gels, powders, precipitates, e.g. alum, or the like. In some situations, it may be desirable to provide for continuous infusion into the host, by employing convenient delivery systems, such as catheters, constant diffusion membranes, pumps, or the like. These formulations and techniques are well known in the literature. Administration may be by injection, for example, intravascular, peritoneally, subcutaneously, subtopically, intradermal patches, inhalation, etc.

The amount of the subject compositions will vary widely depending upon the particular purpose, i.e., prophylaxis or treatment, the manner of administration, the duration of the treatment, the frequency of repetitive treatment and the like. Thus, for the most part, with each composition, the optimal amount used will be determined empirically, but will not differ greatly from similarly formulated peptide vaccines. However, some general considerations can be made concerned with the administration of peptides to a host. To that extent, the peptides generally range from about 0.01 to 10 µg/kg of host, where concentrations will generally range from about 10 µg/ml-1mg/ml. Other additives may be included in the formulations, such as stabilizers, antibiotics, excipients, adjuvants, precipitates for adsorption, slow release additives, etc., or the peptides may be bound to a carrier such as a protein or lipid, e.g. an adjuvant.

A method for treating a host susceptible to HIV comprises administering to the host in an amount sufficient to induce antibodies a peptide compound of this invention. The administration can be repeated on one or more occasions, usually at least two weeks, more usually six weeks, to one year apart or longer. The method is useful in preventing infection and also for inducing antibody production in an infected host as a method of treating the disease.

In a preferred mode for therapeutic treatment of an infected host, the method comprises the following steps. This method may also be used to vaccinate an uninfected host, but is more cumbersome and may not provide significant advantages for prophylactic treatment. In the preferred method, a peptide compound of this invention is administered to the host in an amount sufficient to induce antibodies. The administration of the compound is repeated at intervals of about two weeks to about eight weeks for a period of from about three months to about a year. Thereafter, a different peptide compound is administered at intervals of about two weeks to about eight weeks for a period of about three months to about one year. The successive peptide compound differs from peptide compounds previously administered in the treatment method by having an immunodominant sequence which binds to a subset of T-cells other than any subset bound by peptide compounds previously administered in the treatment method. The peptide compounds may also differ in HIV B-cell epitope sequences from compounds previously administered, but usually will not. The administration of peptide compounds differing in the immunodominant sequence may be continued for the lifetime of the host. A plurality of peptide compounds may be administered as part of the treatment composition.

The following examples are offered by way of illustration and not by way of limitation.

### EXPERIMENTAL

### Example 1

A synthetic peptide is prepared according to the solid phase method of Merrifield et al. The peptide immunogen has the same amino acid sequence as residues 735-752 of gp160 of HIV as reported by Kennedy et al. supra, joined at the carboxy-terminus to the amino-terminus of an immunodominant sequence of lambda cI corresponding to residues 73-94, by itself or further joined to residues 12-26 to provide a response from a broad haplotype spectrum. The sequences are joined together to form a linear peptide.

Lymphocytes are harvested from a patient with AIDS-related complex (ARC) and are cultured. The cultured lymphocytes are divided into two groups. The first group is exposed to 10-100 µg of heat-inactivated HIV. The second group is exposed to 10-100 µg of the peptide compound. Two days later, the supernatant media of the two culture groups is assayed for production of anti-HIV antibodies and HIV. The cultured lymphocytes exposed to heat-inactivated HIV show production of anti-HIV antibodies and HIV. The culture stimulated with the peptide compound shows production of anti-HIV antibodies and substantially no HIV production.

This experiment demonstrates that both groups of cultured lymphocytes are stimulated to produce anti-HIV antibodies. However, unlike exposure to HIV proteins, the peptide compound does not also stimulate production of HIV.

The present peptide compounds stimulate production of antibodies which bind to HIV while minimizing the likelihood of stimulating infected T-cells leading to the production of HIV.

Therefore the peptides can be used to induce protective antibodies in an uninfected host and also offer the advantage that they can be used to stimulate antibody production useful in controlling the progression of the disease in infected hosts.

## Claims (Claims for the following Contracting State(s): AT, BE, CH, DE, FR, GB, IT, LI, LU, NL, SE)

1. A peptide compound comprising:
a first region of at least 6 amino acids, cross-reactive with an HIV B-cell epitope; and
a second region comprising an immunodominant sequence which is capable of stimulating T-cells in conjunction with an HLA;
said immunodominant sequence comprising a sequence of a protein fragment other than of an HIV protein, and said immunodominant sequence comprising at least 5 and not more than 50 amino acids.

2. The peptide compound of Claim 1 wherein said HLA is DR.

3. The peptide compound of Claim 1 or Claim 2 wherein said first region is cross-reactive with a neutralizing epitope.

4. A peptide compound according to any preceding claim wherein said immunodominant sequence comprises a naturally-occurring protein or fragment thereof, and said immunodominant sequence comprises at least 5 and not more than 50 amino acids and not more than about 75 percent of said naturally-occurring protein.

5. A peptide compound according to Claim 4 wherein said immunodominant sequence comprises a fragment of a naturally-occurring protein having at least one mutation wherein said mutation enhances binding of said peptide to said HLA.

6. A peptide compound according to Claim 4 or Claim 5 wherein said HIV B-cell epitope sequence consists of a sequence of from 6 to 30 amino acids of an HIV protein.

7. A peptide compound comprising:
an immunodominant sequence comprising at least 5 and not more than 50 amino acids of an antigen protein other than of an HIV protein restricted by a Class II HLA and able to provide for T-cell stimulation; and
a B-cell epitope comprising at least about 6 amino acids having the same amino acid sequence as consecutive amino acids of an HIV protein and having not more than 3 mutations.

8. The peptide compound according to Claim 7 wherein said HIV protein is gp160.

9. The peptide compound according to Claim 7 wherein said HIV protein is gp120.

10. The peptide compound according to Claim 7 wherein said protein is p17.

11. A peptide composition capable of treating a host susceptible to HIV comprising at least two different peptide compounds in a physiologically suitable medium, each said peptide compound comprising:
a first region of at least 6 amino acids, cross-reactive with an HIV B-cell epitope; and
a second region comprising an immunodominant sequence of a protein other than of an HIV protein which is able to stimulate T-cells in conjunction with an HLA;
with the proviso that when said immunodominant sequence comprises a naturally-occurring protein or fragment thereof, said immunodominant sequence comprises at least 5 amino acids and not more than 36 amino acids and not more than about 75 percent of said naturally-occurring protein;
wherein each said peptide compound differs in said B-cell epitope and/or said immunodominant sequence.

12. A pharmaceutical composition comprising a peptide composition according to any one of the preceding claims.

## Claims (Claims for the following Contracting State(s): ES, GR)

1. A method of making a peptide compound comprising forming it with
a first region of at least 6 amino acids, cross-reactive with an HIV B-cell epitope; and
a second region comprising an immunodominant sequence which is capable of stimulating T-cells in conjunction with an HLA;
said immunodominant sequence comprising a sequence of a protein fragment other than of an HIV protein, and said immunodominant sequence comprising at least 5 and not more than 50 amino acids.

2. The method according to claim 1 wherein said HLA is DR.

3. The method according to claim 1 or claim 2 wherein said first region is cross-reactive with a neutralizing epitope.

4. A method of making a peptide compound according to any preceding claim wherein said immunodominant sequence comprises a naturally-occurring protein or fragment thereof, and said immunodominant sequence comprises at least 5 and not more than 50 amino acids and not more than about 75 percent of said naturally-occurring protein.

5. A method according to claim 4 wherein said immunodominant sequence comprises a fragment of a naturally-occurring protein having at least one mutation wherein said mutation enhances binding of said peptide to said HLA.

6. A method according to claim 4 or claim 5 wherein said HIV B-cell epitope sequence consists of a sequence of from 6 to 30 amino acids of an HIV protein.

7. A method of making a peptide compound comprising making it with
an immunodominant sequence comprising at least 5 and not more than 50 amino acids of other than of an HIV protein restricted by a Class II HLA and able to provide for T-cell stimulation; and
a B-cell epitope comprising at least about 6 amino acids having the same amino acid sequence as consecutive amino acids of an HIV protein and having not more than 3 mutations.

8. The method according to claim 7 wherein said HIV protein is gp160.

9. A method according to claim 7 wherein said HIV protein is gp120.

10. The method according to claim 7 wherein said protein is p17.

11. A method of making a peptide composition capable of treating a host susceptible to HIV comprising at least two different peptide compounds in a physiologically suitable medium, wherein the method comprises forming:
a first region of at least 6 amino acids, cross-reactive with an HIV B-cell epitope; and
a second region comprising an immunodominant sequence of a protein other than of an HIV protein which is able to stimulate T-cells in conjunction with an HLA;
with the proviso that when said immunodominant sequence comprises a naturally-occurring protein or fragment thereof, said immunodominant sequence comprises at least 5 and not more than 36 amino acids and not more than about 75 percent of said naturally-occurring protein;
wherein each said peptide compound differs in said B-cell epitope and/or said immunodominant sequence.

12. A method of making a pharmaceutical composition comprising forming a peptide by a method according to any one of the preceding claims and admixing it with a pharmaceutically acceptable medium.

## Patentansprüche (Patentansprüche für folgende(n) Vertragsstaat(en): AT, BE, CH, DE, FR, GB, IT, LI, LU, NL, SE)

1. Peptidverbindung umfassend:
einen ersten Bereich von zumindest 6 Aminosäuren, der mit einem HIV B-Zellepitop kreuzreaktiv ist; und
einen zweiten Bereich umfassend eine immunodominante Sequenz, die fähig ist, T-Zellen in Verbindung mit einem HLA zu stimulieren;
wobei die genannte immunodominante Sequenz eine Sequenz eines Proteinfragments mit Ausnahme eines HIV-Proteins umfaßt und die genannte immunodominante Sequenz zumindest 5 und nicht mehr als 50 Aminosäuren umfaßt.

2. Peptidverbindung nach Anspruch 1, worin das genannte HLA DR ist.

3. Peptidverbindung nach Anspruch 1 oder Anspruch 2, worin der genannte erste Bereich mit einem neutralisierenden Epitop kreuzreaktiv ist.

4. Peptidverbindung nach einem der vorhergehenden Ansprüche, worin die genannte immunodominante Sequenz ein natürlich vorkommendes Protein oder Fragment davon umfaßt und die genannte immunodominante Sequenz zumindest 5 und nicht mehr als 50 Aminosäuren und nicht mehr als etwa 75% des genannten natürlich vorkommenden Proteins umfaßt.

5. Peptidverbindung nach Anspruch 4, worin die genannte immunodominante Sequenz ein Fragment eines natürlich vorkommenden Proteins mit zumindest einer Mutation umfaßt, worin die genannte Mutation die Bindung des genannten Peptids an das genannte HLA verbessert.

6. Peptidverbindung nach Anspruch 4 oder Anspruch 5, worin die genannte HIV B-Zellepitopsequenz aus einer Sequenz von 6 bis 30 Aminosäuren eines HIV-Proteins besteht.

7. Peptidverbindung umfassend:
eine immunodominante Sequenz umfassend zumindest 5 und nicht mehr als 50 Aminosäuren eines Antigenproteins mit Ausnahme eines HIV-Proteins, eingeschränkt durch ein HLA der Klasse II und fähig, T-Zellstimulierung zu bewirken; und
ein B-Zellepitop umfassend zumindest etwa 6 Aminosäuren mit der gleichen Aminosäuresequenz wie aufeinanderfolgende Aminosäuren eines HIV-Proteins und mit nicht mehr als 3 Mutationen.

8. Peptidverbindung nach Anspruch 7, worin das genannte HIV-Protein gp 160 ist.

9. Peptidverbindung nach Anspruch 7, worin das genannte HIV-Protein gp 120 ist.

10. Peptidverbindung nach Anspruch 7, worin das genannte Protein p17 ist.

11. Peptidzusammensetzung, die geeignet ist, einen für HIV anfälligen Wirt zu behandeln, umfassend zumindest zwei unterschiedliche Peptidverbindungen in einem physiologisch geeigneten Medium, wobei jede genannte Peptidverbindung umfaßt:
einen ersten Bereich von zumindest 6 Aminosäuren, der mit einem HIV B-Zellepitop kreuzreaktiv ist; und
einen zweiten Bereich, der eine immunodominante Sequenz eines Proteins mit Ausnahme eines HIV-Proteins umfaßt, die fähig ist, T-Zellen in Verbindung mit einem HLA zu stimulieren;
mit der Maßgabe, daß dann, wenn die genannte immunodominante Sequenz ein natürlich vorkommendes Protein oder Fragment davon umfaßt; die genannte immunodominante Sequenz zumindest 5 Aminosäuren und nicht mehr als 36 Aminosäuren und nicht mehr als etwa 75 % des genannten natürlich vorkommenden Proteins umfaßt;
wobei sich jede genannte Peptidverbindung im B-Zellepitop und/oder der genannten immunodominanten Sequenz unterscheidet.

12. Pharmazeutische Zusammensetzung umfassend eine Peptidzusammensetzung nach einem der vorhergehenden Ansprüche.

## Patentansprüche (Patentansprüche für folgende(n) Vertragsstaat(en): ES, GR)

1. Verfahren zum Herstellen einer Peptidverbindung umfassend ihr Bilden mit:
einem ersten Bereich von zumindest 6 Aminosäuren, der mit einem HIV B-Zellepitop kreuzreaktiv ist; und
einem zweiten Bereich umfassend eine immunodominante Sequenz, die fähig ist, T-Zellen in Verbindung mit einem HLA zu stimulieren;
wobei die genannte immunodominante Sequenz eine Sequenz eines Proteinfragments mit Ausnahme eines HIV-Proteins umfaßt und die genannte immunodominante Sequenz zumindest 5 und nicht mehr als 50 Aminosäuren umfaßt.

2. Verfahren nach Anspruch 1, worin das genannte HLA DR ist.

3. Verfahren nach Anspruch 1 oder Anspruch 2, worin der genannte erste Bereich mit einem neutralisierenden Epitop kreuzreaktiv ist.

4. Verfahren zum Herstellen einer Peptidverbindung nach einem der vorhergehenden Ansprüche, worin die genannte immunodominante Sequenz ein natürlich vorkommendes Protein oder Fragment davon umfaßt und die genannte immunodominante Sequenz zumindest 5 und nicht mehr als 50 Aminosäuren und nicht mehr als etwa 75% des genannten natürlich vorkommenden Proteins umfaßt.

5. Verfahren nach Anspruch 4, worin die genannte immunodominante Sequenz ein Fragment eines natürlich vorkommenden Proteins mit zumindest einer Mutation umfaßt, worin die genannte Mutation die Bindung des genannten Peptids an das genannte HLA verbessert.

6. Verfahren nach Anspruch 4 oder Anspruch 5, worin die genannte HIV B-Zellepitopsequenz aus einer Sequenz von 6 bis 30 Aminosäuren eines HIV-Proteins besteht.

7. Verfahren zum Herstellen einer Peptidverbindung umfassend ihr Herstellen mit:
einer immunodominanten Sequenz umfassend zumindest 5 und nicht mehr als 50 Aminosäuren eines Antigenproteins mit Ausnahme eines HIV-Proteins, eingeschränkt durch ein HLA der Klasse II und fähig, T-Zellstimulierung zu bewirken; und
einem B-Zellepitop umfassend zumindest etwa 6 Aminosäuren mit der gleichen Aminosäuresequenz wie aufeinanderfolgende Aminosäuren eines HIV-Proteins und mit nicht mehr als 3 Mutationen.

8. Verfahren nach Anspruch 7, worin das genannte HIV-Protein gp 160 ist.

9. Verfahren nach Anspruch 7, worin das genannte HIV-Protein gp 120 ist.

10. Verfahren nach Anspruch 7, worin das genannte Protein p 17 ist.

11. Verfahren zum Herstellen einer Peptidzusammensetzung, die geeignet ist, einen für HIV anfälligen Wirt zu behandeln, umfassend zumindest zwei unterschiedliche Peptidverbindungen in einem physiologisch geeigneten Medium, worin das Verfahren das Bilden:
eines ersten Bereichs von zumindest 6 Aminosäuren, der mit einem HIV B-Zellepitop kreuzreaktiv ist; und
eines zweiten Bereichs, der eine immunodominante Sequenz eines Proteins mit Ausnahme eines HIV-Proteins umfaßt, die fähig ist, T-Zellen in Verbindung mit einem HLA zu stimulieren;
mit der Maßgabe umfaßt, daß dann, wenn die genannte immunodominante Sequenz ein natürlich vorkommendes Protein oder Fragment davon umfaßt, die genannte immunodominante Sequenz zumindest 5 Aminosäuren und nicht mehr als 36 Aminosäuren und nicht mehr als etwa 75% des genannten natürlich vorkommenden Protein umfaßt;
wobei sich jede genannte Peptidverbindung im B-Zellepitop und/oder der genannten immunodominanten Sequenz unterscheidet.

12. Verfahren zum Herstellen einer pharmazeutischen Zusammensetzung umfassend das Bilden einers Peptids durch ein Verfahren nach einem der vorhergehenden Ansprüche und sein Vermischen mit einem pharmazeutisch akzeptablen Medium.

## Revendications (Revendications pour l'(les) Etat(s) contractant(s) suivant(s): AT, BE, CH, DE, FR, GB, IT, LI, LU, NL, SE)

1. Composé peptidique comprenant :
une première région d'au moins 6 acides aminés, réagissant avec un épitope d'une cellule B de VIH; et
une seconde région comprenant une séquence immunodominante qui est capable de stimuler les cellules T en conjonction avec HLA;
ladite séquence immunodominante comprenant une séquence d'un fragment de protéine ou autre qu'une protéine de VIH et ladite séquence immunodominante comprenant au moins 5 et pas plus de 50 acides aminés.

2. Composé peptidique de la revendication 1 où ledit HLA est DR.

3. Composé peptidique de la revendication 1 ou la revendication 2, où ladite première région réagit avec un épitope neutralisant.

4. Composé peptidique selon l'une quelconque des revendications précédentes où ladite séquence immunodominante comprend une protéine naturelle ou son fragment, et ladite séquence immunodominante se compose d'au moins 5 et de pas plus de 50 acides aminés et de pas plus d'environ 75 pourcent de ladite protéine naturelle.

5. Composé peptidique selon la revendication 4, où ladite séquence immunodominante se compose d'un fragment d'une protéine naturelle ayant au moins une mutation où ladite mutation améliore la liaison dudit peptide audit HLA.

6. Composé peptidique selon La revendication 4 ou la revendication 5, où ladite séquence de l'épitode de la cellule B de VIH se compose d'une séquence de 6 à 30 acides aminés d'une protéine de VIH.

7. Composé peptidique comprenant :
une séquence immunodominante comprenant au moins 5 et pas plus de 50 acides aminés d'une protéine d'un antigène autre qu'une protéine de VIH restreinte par HLA de la classe II et pouvant permettre la stimulation de cellules T, et
un épitope de cellule B comprenant au moins environ 6 acides aminés ayant la même séquence d'acides aminés que des acides aminés consécutifs d'une protéine de VIH et n'ayant pas plus de 3 mutations.

8. Composé peptidique selon la revendication 7 où ladite protéine de VIH est gp160.

9. Composé peptidique selon la revendication 7, où ladite protéine de VIH est gp120.

10. Composé peptidique selon la revendication 7, où ladite protéine est p17.

11. Composé peptidique capable de traiter un hôte sensible à VIH comprenant au moins deux composés peptidiques différents dans un milieu physiologiquement approprié, chacun composé peptidique comprenant :
une première région d'au moins 6 acides aminés réagissant avec un épitope d'une cellule B de VIH; et
une seconde région comprenant une séquence immunodominante d'une protéine autre qu'une protéine de VIH qui peut stimuler les cellules en association avec HLA;
à condition que lorsque ladite séquence immunodominante comprend une protéine naturelle ou son fragment ladite séquence immunodominante comprenne au moins 5 acides aminés et pas plus de 36 acides aminés et pas plus d'environ 75% de ladite protéine naturelle.
où chaque composé peptidique diffère par ledit épitope de cellule B et/ou ladite séquence immunodominante.

12. Composition pharmaceutique comprenant une composition d'un peptide selon l'une quelconque des revendications précédentes.

## Revendications (Revendications pour l'(les) Etat(s) contractant(s) suivant(s): ES, GR)

1. Méthode de fabrication d'un composé peptidique comprenant sa formation avec une première région d'au moins 6 acides aminés, réagissant avec un épitope d'une cellule B de VIH; et
une seconde région comprenant une séquence immunodominante qui est capable de stimuler les cellules T en conjonction avec HLA;
ladite séquence immunodominante comprenant une séquence d'un fragment de protéine ou autre qu'une protéine de VIH et ladite séquence immunodominante comprenant au moins 5 et pas plus de 50 acides aminés.

2. Méthode selon la revendication 1 où ledit HLA est DR.

3. Méthode selon la revendication 1 ou 2, où ladite première région réagit avec un épitope neutralisant.

4. Méthode de fabrication d'un composé peptidique selon l'une quelconque des revendications précédentes où ladite séquence immunodominante comprend une protéine se produisant naturellement ou son fragment, et où ladite séquence immunodominante se compose d'au moins 5 et de pas plus de 50 acides aminés et de pas plus d'environ 75 pourcent de ladite protéine se produisant naturellement.

5. Méthode selon la revendication 4, où ladite séquence immunodominante comprend un fragment d'une protéine se produisant naturellement ayant au moins une mutation ou ladite mutation améliore la liaison dudit peptide audit HLA.

6. Méthode selon la revendication 4 ou 5, où ladite séquence de l'épitope dos cellules B de VIH se compose d'une séquence de 6 à 30 acides aminés d'une protéine de VIH.

7. Méthode de fabrication d'un composé peptidique comprenant sa fabrication avec
une séquence immunodominante comprenant au moins 5 et pas plus de 50 acides aminés d'une protéine autre qu'une protéine de VIH restreinte par HLA de la classe II et pouvant permettre la stimulation de cellules T, et
un épitope de cellule B comprenant au moins environ 6 acides aminés ayant la même séquence d'acides aminés que des acides aminés consécutifs d'une protéine de VIH et n'ayant pas plus de 3 mutations.

8. Méthode selon la revendication 7 où ladite protéine de VIH est gp160.

9. Méthode selon la revendication 7, où ladite protéine de VIH est gp120.

10. Méthode selon la revendication 7, où ladite protéine est p17.

11. Méthode de fabrication d'une composition de peptides capable de traiter un hôte sensible à VIH comprenant au moins deux composés peptidiques différents dans un milieu physiologiquement approprié, où la méthode comprend la formation de :
une première région d'au moins 6 acides aminés réactive en croisé avec un épitope de cellules B de VIH; et
une seconde région comprenant une séquence immunodominante d'une protéine antre qu'une protéine de VIH qui peut stimuler les cellules T en association avec HLA;
à condition que lorsque ladite séquence immunodominante comprend une protéine se produisant naturellement ou son fragment, ladite séquence immunodominante comprend au moins 5 acides aminés et pas plus de 36 acides aminés et pas plus d'environ 75% de ladite protéine se produisant naturellement;
où chaque composé peptidique diffère par ledit épitope de cellules B et/ou ladite séquence immunodominante.

12. Méthode de fabrication d'une composition pharmaceutique comprenant la formation d'un peptide par une méthode selon l'une quelconque des revendications précédentes et son mélange avec un milieu pharmaceutiquement acceptable.
